# EUROPÄISCHE PATENTSCHRIFT

(11) **EP 2 832 379 B1**
(45) Veröffentlichungstag und Bekanntmachung des Hinweises auf die Patenterteilung: **22.07.2015**
(21) Anmeldenummer: 13178696.4
(22) Anmeldetag: 31.07.2013
(51) Int. Cl.: A61L 27/36

(54) **Verfahren zur Aufbereitung von biologischem Gewebe**
Method for the treatment of biological tissue
Procédé de préparation de tissus biologiques

(43) Veröffentlichungstag der Anmeldung: 04.02.2015
(73) Patentinhaber: Biotronik AG, 8180 Bülach (CH)
(72) Erfinder: Rzany, Alexander, 90449 Nürnberg (DE); Erdbrügger, Wilhelm, 78467 Konstanz (DE)
(74) Vertreter: Galander, Marcus

(56) Entgegenhaltungen:
- WO-A1-02/49681
- WO-A1-2004/052417

## Beschreibung

Die vorliegende Erfindung betrifft ein Verfahren zur Aufbereitung von Gewebe für medizinische Anwendungen, insbesondere von Gewebe zur Verwendung für eine künstliche Herzklappe, wobei das Verfahren zumindest folgende Schritte umfasst:
- Dezellularisierung des Gewebes mittels eines geeigneten Detergens und nachfolgende
- Vernetzung der Kollagenfasern des Gewebes mittels eines geeigneten Vernetzungsmittels.

Die Erfindung wird im Folgenden am Beispiel eines Verfahrens zur Aufbereitung von Gewebe zur Verwendung für eine künstliche Herzklappe beschrieben. Die vorliegende Erfindung ist zwar zur Aufbereitung derartigen Gewebes besonders geeignet, jedoch nicht auf diese Anwendung beschränkt. Die vorliegende Erfindung ist auch für die Aufbereitung von Blutgefäßen, Knochenknorpeln, Bändern oder dergleichen anwendbar.

Die Erfindung betrifft ferner eine Verwendung für mindestens eine Lipopeptide enthaltende Lösung.

Es gibt grundsätzlich zwei verschiedene Arten von Herzklappenprothesen: Prothesen mit mechanischen Klappen, welche künstlich, zumeist aus mit pyrolytischem Kohlenstoff beschichteten Graphit, hergestellt werden, und Prothesen mit Klappen aus biologischem Gewebe, zumeist Herzbeutelgewebe, welches zumeist aus tierischen Quellen (z.B. Schwein oder Rind) stammt. Die aus dem biologischen Gewebe geformte Herzklappe wird zumeist in einem Grundkörper (z.B. ein festes Kunststoffgerüst oder ein selbstexpandierender Stent) befestigt und dieser wird an der Position der natürlichen Klappe implantiert. Die vorliegende Erfindung beschreibt ein Verfahren zur Aufbereitung eines derartigen Gewebes zum Einsatz in einer Herzklappenprothese, die an Stelle einer natürlichen Herzklappe implantiert werden soll.

Das Ausgangsgewebe muss vor der Implantation gründlich gereinigt und aufbereitet werden. Dabei wird das Gewebe möglichst so modifiziert, dass es vom Körper nicht als Fremdgewebe erkannt wird, nicht verkalkt und eine möglichst lange Lebensdauer hat. Im Wesentlichen umfasst ein derartiges Verfahren zur Aufbereitung von Gewebe mindestens zwei Hauptschritte mit mehreren zwischengeschalteten Spülprozessen.

Der erste wesentliche Aufbereitungsschritt ist die sogenannte Dezellularisierung des Gewebes. In diesem Schritt werden Zellmembranen, intrazelluläre Proteine, Zellkerne und andere Zellbestandteile möglichst vollständig aus dem Gewebe entfernt, um eine möglichst reine extrazelluläre Matrix zu erhalten. Im Gewebe verbleibende Zellen und Zellbestandteile wären insbesondere potente Kristallisationskeime für eine unerwünschte Kalzifizierung des biologischen Implantatmaterials. Die Dezellularisierung als Waschschritt soll dabei möglichst so schonend durchgeführt werden, dass die Struktur und die Kollagenfasern in der extrazellulären Matrix möglichst unbeeinflusst bleiben, während andererseits gründlich alle darin enthaltenen Zellen aus dem Gewebe entfernt werden.

Der zweite wesentliche Aufbereitungsschritt besteht in einer Vernetzung, insbesondere der Kollagenfasern, des Gewebes. Nach der Dezellularisierung sind möglichst alle Zellbestandteile aus dem Gewebe entfernt und das biologische Material besteht fast ausschließlich aus der extrazellulären Matrix. Bei Herzbeutelgewebe wird die extrazelluläre Matrix dabei vorwiegend aus Kollagenfasern gebildet. Um ein biologisches Material mit möglichst optimalen mechanischen Eigenschaften zu erreichen und Abwehrreaktionen des empfangenden Körpers zu verhindern, werden die Kollagenfasern mittels eines geeigneten Vernetzungsmittels durch den Einbau chemischer Bindungen quervernetzt. Das Vernetzungsmittel bindet an die Aminogruppen der Kollagenfasern an und bildet chemisch stabile Verbindungen zwischen Kollagenfasern. So entsteht aus dem dreidimensional angeordneten Kollagenfasern ein langzeitstabiles biologisches Material, welches zudem nicht mehr als biologisches Fremdmaterial erkannt wird. Durch die dreidimensionale Vernetzung bzw. Verknüpfung der einzelnen Kollagenfasern über das Vernetzungsmittel werden die Stabilität und die Beanspruchbarkeit des Gewebes deutlich erhöht. Dies ist insbesondere bei einem Einsatz als Gewebe einer Herzklappe entscheidend, wo das Gewebe über dem Sekundentakt als Klappe öffnen und schließen muss.

WO 02/49681 ein Verfahren zur Dezellularisierung von biologischem Material mit Hilfe von biologischen Detergentien, bei dem das Fremdmaterial zunächst mit Gallensäure und danach mit Alkohol behandelt wird und beiden Schritten ein Spülvorgang nachgeschaltet ist.

Ein alternatives Verfahren zur Aufbereitung von biologischem Gewebe wird in WO 2004/052417 beschrieben. In diesem Verfahren nach dem Stand der Technik wird das Gewebe mit einer 1-2%igen Desoxycholsäure Lösung dezellularisiert. Nach mehreren Spülschritten erfolgt die Konditionierung des Gewebes in einer Lösung, die ein zyklisches Lipopeptid enthält. Dabei wird insbesondere das zyklische Lipopeptid Surfactin als Konditionierungsmittel nach der erfolgten Dezellularisierung und vor einer Wiederbesiedelung mit Zellen verwendet. In diesem alternativen Verfahren nach dem Stand der Technik erfolgt keine Quervernetzung der Kollagenfasern durch ein geeignetes Vernetzungsmittel. Nach der Konditionierung wird das Gewebe mit natürlichen Zellen besiedelt.

WO 2011/109433 offenbart ein Verfahren zur Aufbereitung von biologischem Gewebe, wobei destilliertes Wasser zur Dezellularisierung verwendet wird. Glutaraldehyd wirkt in einem nachgelagerten Schritt als Vernetzungsmittel.

WO 2005/118014 offenbart die Verwendung eines ersten ionischen Detergens und eines zweiten nichtionischen Detergens zur Dezellularisierung. Hierbei werden als erstes ionisches Detergens, bevorzugt ein anionisches Detergens wie Natriumdodecylsulfat oder Natriumdodecylsulfonat vorgeschlagen. Alternativ können als erstes Detergens Gallensäuren wie Natriumcholat oder Natriumdesoxycholat verwendet werden. Das zweite Detergens ist elektrisch neutral geladen, wie beispielsweise Polyethylenglykol enthaltendes Detergens.

Der vorliegenden Erfindung liegt daher die Aufgabe zugrunde, ein Verfahren zur Aufbereitung von biologischen Gewebe derart auszugestalten, dass Zellbestandteile gründlich aber schonend vom Gewebe entfernt werden, so eine anschließende Vernetzung ein mechanisch stabiles und langlebiges Gewebe erzeugt, welches insbesondere zu einem Einsatz als Gewebe einer künstlichen Herzklappe geeignet ist.

Der vorliegenden Erfindung liegt ferner die Aufgabe zugrunde, eine neue Verwendung für Lösungen zu finden, die mindestens ein Lipopeptid enthalten.

Die gestellte Aufgabe wird verfahrensseitig durch die Merkmalskombination des Anspruchs 1 gelöst. Die gestellte Aufgabe wird verwendungsseitig durch die Merkmalskombination des Anspruches 6 gelöst. Vorteilhafte Ausgestaltungen der Erfindungen werden in den Unteransprüchen ausgeführt.

Die Grundidee der Erfindung ist der Einsatz von Lipopeptiden als Detergens zur Dezellularisierung. Gemäß der erfinderischen Idee werden β-Hydroxy-Fettsäure- oder β-Amino-Fettsäure-haltige Peptide, Lipopeptide, nicht zur Konditionierung sondern als Detergens zur Dezellularisierung verwendet. Überraschenderweise hat sich gezeigt, dass die Lipopeptide hervorragende Ergebnisse bei der Dezellularisierung von Gewebe zeigen. Das Gewebe wird deutlich schonender von Zellbestandteilen befreit. Die Struktur der extrazellulären Matrix bleibt bei der Verwendung eines erfindungsgemäßen Detergens zur Dezellularisierung deutlich besser erhalten als bei einem Detergens nach dem Stand der Technik. Das erfindungsgemäße Detergens enthält somit mindestens ein Lipopeptid mit amphiphilen Eigenschaften, bestehend aus einer hydrophilen Grundstruktur und einer hydrophoben Seitenkette. Dadurch lässt sich durch den nachfolgenden Vernetzungsschritt ein Gewebe erzielen, was gegenüber dem Stand der Technik deutlich verbesserte mechanische Eigenschaften aufweist und sich somit insbesondere zur Verwendung in einer Herzklappenprothese eignet.

Besonders bevorzugt enthält das Detergens zur Dezellularisierung ein zyklisches Lipoheptapeptid, insbesondere Surfactin. In dieser bevorzugten Ausgestaltung der Erfindung wird ein Surfactin enthaltendes Detergens zur Dezellularisierung verwendet. Insbesondere enthält das Detergens Surfactin mit einer zyklischen Struktur wie nachfolgend wiedergegeben:

(CH3)2-CH-(CH2)n→CH-CH2-CO→L-Glu→L-Leu→D-Leu→L-Val→L-Asp→D-Leu→L-Leu→O

Dabei ist n=10-12 und Glu, Leu, Val, Asp stehen für die Aminosäuren Glutaminsäure, Leucin, Valin und Asparginsäure.

Ebenso vorteilhaft sind Lipopeptide wie Daptomycin, Caspofungin, Arthrofactin, Echinocandine, Iturine, Syringomycine, Syringopeptide und/oder Polymyxine. Zweckmäßigerweise enthält das Detergens mindestens ein Lipopeptid ausgewählt aus der Liste: Surfactin, Daptomycin, Caspofungin, Arthrofactin oder der Gruppe der Echinocandine, Iturine, Syringomycine, Syringopeptide, Polymyxine.

Das Vernetzungsmittel enthält bevorzugt Glutaraldehyd. In alternativen Ausgestaltungen der Erfindung enthält das Vernetzungsmittel Carbodiimid, Formaldehyd, Glutaraldehyd-Acetale, Acyl-Azide, Cyanimid, Genepin, Tannin, Pentagalloyl-Glukose, Phytat, Proanthocyanidin, Reuterin und/oder Epoxidverbindungen.

Vorteilhafterweise wird das Gewebe vor und besonders bevorzugt nach der Dezellularisierung zumindest einmal, bevorzugt mehrmals, mit einem geeigneten Lösungsmittel, insbesondere einer gepufferten Salzlösung und/oder einer Alkohollösung, gespült.

Besonders vorteilhaft sind dabei gepufferte Natriumchloridlösungen und/oder eine Ethanollösung.

Besonders bevorzugt besteht das Detergens aus einer Pufferlösung, besonders bevorzugt einer Phosphatpufferlösung, zweckmäßigerweise bei pH 7.4, die das Lipopeptid, insbesondere Surfactin, mit einer Konzentration von 100 mg/l bis 2000 mg/l, bevorzugt 500 mg/l bis 700 mg/l, besonders bevorzugt von 600 mg/l, enthält. Dabei ist der Einsatz von Dulbecco's Phosphat gepufferter Salzlösung (DPBS) ohne Kalzium und Magnesium als Trägerlösung für das Detergens Surfactin besonders vorteilhaft. Andere biologische Pufferlösungen, wie zum Beispiel Tris(hydroxymethyl)-aminomethan (TRIS)- oder 2-(4-(2-Hydroxyethyl)- 1-piperazinyl)-ethansulfonsäure (HEPES)-gepufferte Lösungen, sind ebenfalls zweckmäßig.

Die vorliegende Erfindung betrifft ferner die Verwendung einer Lösung, die mindestens ein Lipopeptid mit amphiphilen Eigenschaften, bestehend aus einer hydrophilen Grundstruktur und einer hydrophoben Seitenkette, enthält, als Detergens zur Dezellularisierung von biologischem Gewebe, insbesondere von biologischem Gewebe für Herzklappenprothesen. Erfindungsgemäß wird eine Lösung, die mindestens ein Lipopeptid enthält, nicht zur Konditionierung sondern zur Dezellularisierung und Reinigung von biologischem Gewebe verwendet.

Es hat sich überraschender Weise gezeigt, dass Lipopeptide enthaltende Lösungen biologisches Gewebe gründlich aber schonend dezellularisieren.

Besonders vorteilhaft ist die Verwendung einer mindestens Surfactin, Daptomycin, Caspofungin, Arthrofactin , ein Echinocandin, ein Iturin, ein Syringomycin, ein Syringopeptid und/oder ein Polymyxin enthaltenden Lösung als Detergens zur Dezellularisierung von biologischem Gewebe.

Zweckmäßig ist dabei, wenn Surfactin, Daptomycin, Caspofungin, Arthrofactin und/oder ein Echinocandin, Iturin, Syringomycin, Syringopeptid, Polymyxin in einer Pufferlösung, insbesondere einer Phosphatpufferlösung, gelöst ist. Zweckmäßig sind auch TRIS- oder HEPES-gepufferte Lösungen.

Die vorliegende Erfindung stellt insbesondere ein Verfahren zur Aufbereitung von biologischem Gewebe zur Verfügung, welches eine gründliche und zuverlässige Dezellularisierung gewährleistet, welche gleichzeitig derart gewebeschonend durchgeführt wird, dass die mechanischen Eigenschaften des Gewebes nach Dezellularisierung und Vernetzung deutlich gegenüber dem Stand der Technik verbessert werden.

Das erfindungsgemäße Verfahren zur Aufbereitung von biologischem Gewebe, insbesondere zur Aufbereitung von biologischem Gewebe zur Verwendung in einer Herzklappenprothese, minimiert das Risiko einer Kalzifizierung des Gewebes (und damit der Prothese) im klinischen Einsatz. Durch das erfindungsgemäß verwendete Detergens zur Dezellularisierung werden maßgeblich die Eigenschaften des Gewebes positiv beeinflusst. Ein nach dem erfindungsgemäßen Verfahren aufbereitetes Gewebe zeigt eine deutlich verbesserte mechanische Belastbarkeit.

Im Folgenden soll die Erfindung anhand der Ausführungsbeispiele in den Figuren näher erläutert und mit einem Verfahren nach dem Stand der Technik verglichen werden.

In einem Ausführungsbeispiel der Erfindung wird ein biologisches Gewebe aus porcinem Herzbeutelgewebe durch mechanische Entfernung von anhaftendem Fremdgewebe und anschließendem Spülen in isotonischer Kochsalzlösung (Fa. Fresenius-Kabi) für 20 Stunden gewonnen. Dieses Gewebe wird einer Dezellularisierung mit einem Detergens, bestehend aus einer DPBS-Lösung ohne Kalzium/Magnesium (Fa. Lonza; DPBS w/o Ca++/Mg++; Art.-Nr. 17-512) und Surfactin (Fa. Sigma-Aldrich, Surfactin from Bacillus subtilis, Art.-Nr. F3523) in einer Konzentration von 600 mg/l, unterzogen.

Das obige Ausführungsbeispiel nach der vorliegenden Erfindung wird mit zwei Detergenzien nach dem Stand der Technik verglichen.

Im ersten Beispiel nach dem Stand der Technik wird das biologische Gewebe einer Dezellularisierung mit einem Detergens unterzogen, welches Natriumdodecylsulfat (SDS; Fa. Sigma-Aldrich, Art.-Nr. L3771) in einer Konzentration von 5 g/l enthält. Als Lösungsmittel kommt hierbei ebenfalls DPBS-Lösung ohne Kalzium/Magnesium (Fa. Lonza; DPBS w/o Ca++/Mg++; Art.-Nr. 17-512) zum Einsatz.

In einem zweiten Beispiel nach dem Stand der Technik wird das biologische Gewebe einer Dezellularisierung mit einem Detergens unterzogen, welches Desoxycholsäure (DCA; Fa. Sigma-Aldrich, Art.-Nr. D6750) in einer Konzentration von 10 g/l enthält. Als Lösungsmittel kommt hierbei isotonische Kochsalzlösung (Fa. Fresenius-Kabi) zum Einsatz.

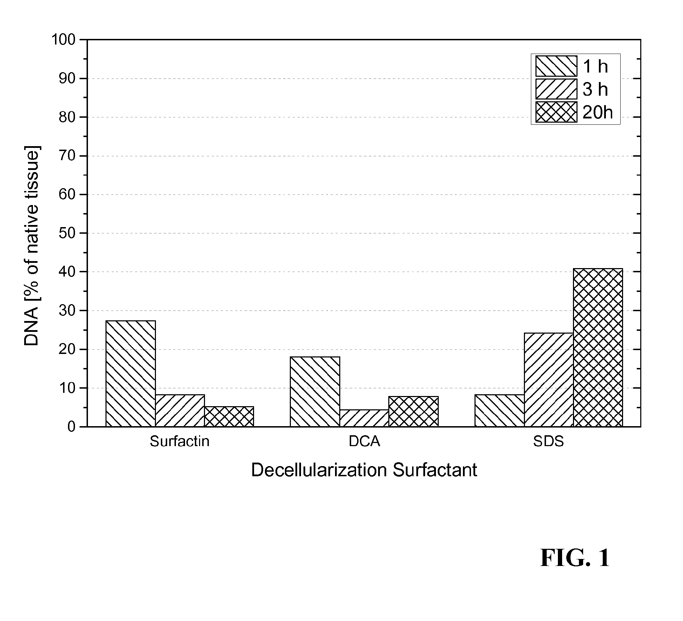

Figur 1 zeigt den Vergleich des DNA-Gehaltes nach Dezellularisierung zwischen dem erfindungsgemäßen Ausführungsbeispiel und den beiden Beispielen nach dem Stand der Technik. Auf der Ordinate in Figur 1 ist der DNA-Gehalt des Herzbeutelgewebes nach Dezellularisierung in % des ursprünglichen DNA-Gehaltes vor der Dezellularisierung aufgetragen. Aufgetragen wurde der DNA-Gehalt jeweils nachdem sich das biologische Gewebe 1 Stunde, 3 Stunden und 20 Stunden in der jeweiligen Waschlösung befunden hat.

Der DNA-Gehalt bildet ein direktes Maß für die Entfernung zellulärer Bestandteile aus dem biologischen Gewebe.

Mit Hilfe des DCA-haltigen Detergens zur Dezellularisierung lässt sich nach drei Stunden der DNA-Gehalt auf ca. 4% reduzieren. Wie in Figur 1 erkennbar, lässt sich der DNA-Gehalt nach 20 Stunden in dem Surfactin-haltigen Detergens des Ausführungsbeispieles der Erfindung auf einen ähnlichen Wert reduzieren. Der mit Surfactin innerhalb 20 Stunden erreichte Dezellularisierungsgrad für Herzbeutelgewebe entspricht dem von Desoxycholsäure. Die Werte des DNA-Gehaltes für das SDS-haltige Detergens sind hier nur bedingt vergleichbar, da SDS eine sehr starke Strukturänderung von Proteinen bewirkt und bei deutlich sichtbarer Dezellularisierung die Nachweismethodik auf DNA massiv beeinträchtigt.

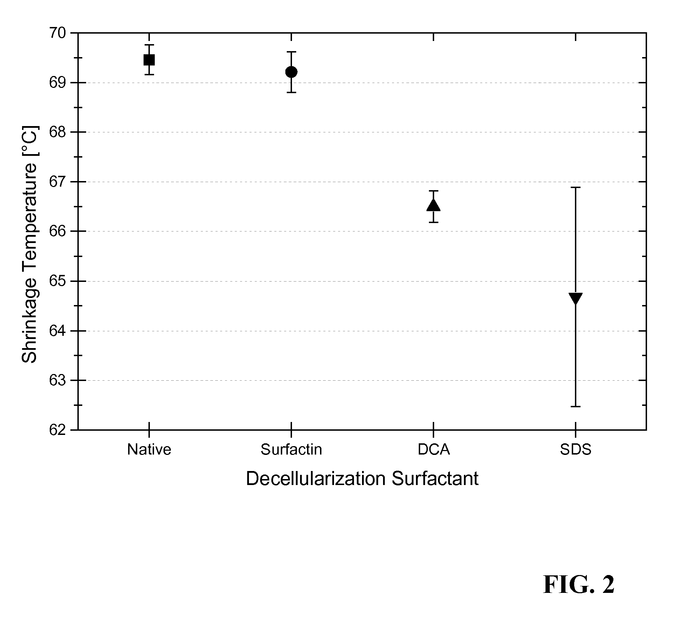

Die gravierenden Vorteile des erfindungsgemäßen Verfahrens gegenüber Dezellularisierungsprozessen nach dem Stand der Technik zeigen sich in den anschließenden Figuren 2 bis 3d.

Figur 2 zeigt auf der Ordinate (vergrößerte Skala, Nullpunkt nicht abgebildet) die Schrumpfungstemperatur (shrinkage temperature) des dezellularisierten Gewebes nach Behandlung mit den genannten drei Detergenzien im Vergleich zur Schrumpfungstemperatur des nativen Gewebes.

Aufgrund des dominierenden Anteils von Kollagen in der extrazellulären Matrix von Herzbeutelgewebe ist die Schrumpfungstemperatur die Temperatur, bei der das Protein Kollagen thermisch denaturiert, d.h. irreversibel seine räumliche Struktur verändert. Infolge der Strukturänderung der Kollagenmoleküle kommt es zu massiven irreversiblen Strukturänderungen im Gewebe, das bei Erreichen der Schrumpfungstemperatur deutlich sichtbar kleiner wird.

Experimentell bestimmt wurde die Schrumpfungstemperatur mittels Differential Scanning Calorimetry (DSC). Bei dieser Methode wird die Temperatur der zu vermessenden Probe linear mit der Zeit erhöht und der Wärmefluss in die bzw. aus der Probe bezüglich einer Referenzprobe vermessen. Kommt es zu thermodynamischen Prozessen in der Probe, z.B. der irreversiblen Strukturänderung des Kollagens, tritt im gemessenen Thermogramm ein deutlicher Peak bei der Schrumpfungstemperatur auf. Die Höhe der Schrumpfungstemperatur ist ein direkter Indikator für die Stabilität der räumlichen Struktur der Kollagenmoleküle. Eine möglichst geringe Veränderung gegenüber dem Zustand in nativem Gewebe ist deshalb ein direkter Beweis auf molekularer Ebene für die deutlich schonendere Dezellularisierung durch Surfactin.

Wie Figur 2 deutlich zeigt, ist die Schrumpfungstemperatur des Herzbeutelgewebes nach einer Dezellularisierung gemäß dem Ausführungsbeispiel der Erfindung nahezu identisch der Schrumpfungstemperatur des unbehandelten nativen Herzbeutelgewebes. Die Dezellularisierung nach den beiden Ausführungsbeispielen des Standes der Technik mit DCA und SDS führen dagegen zu einer deutlich um 3°C bzw. 5°C reduzierten Schrumpfungstemperatur und damit einer deutlich beeinträchtigten Gewebestruktur. Die mechanischen Eigenschaften des nativen biologischen Gewebes und des Gewebes nach erfindungsgemäßer Dezellularisierung sind daher sehr ähnlich. Mit Hilfe des erfindungsgemäßen Verfahrens erfolgt die Dezellularisierung somit nachweislich sehr schonend.

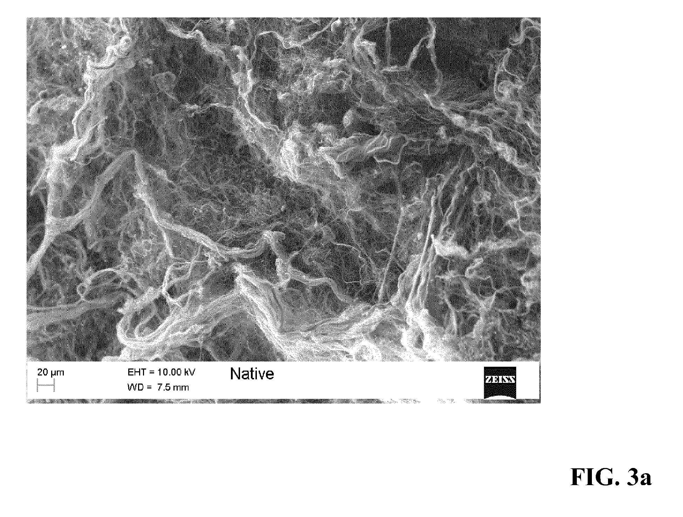

Die unterschiedliche Beeinträchtigung der Gewebestruktur zeigen auch die, in den Figuren 3a-d dargestellten, elektronenmikroskopischen Aufnahmen des nativen Gewebes bzw. des Gewebes nach Dezellularisierung mit den obengenannten Detergenzien.

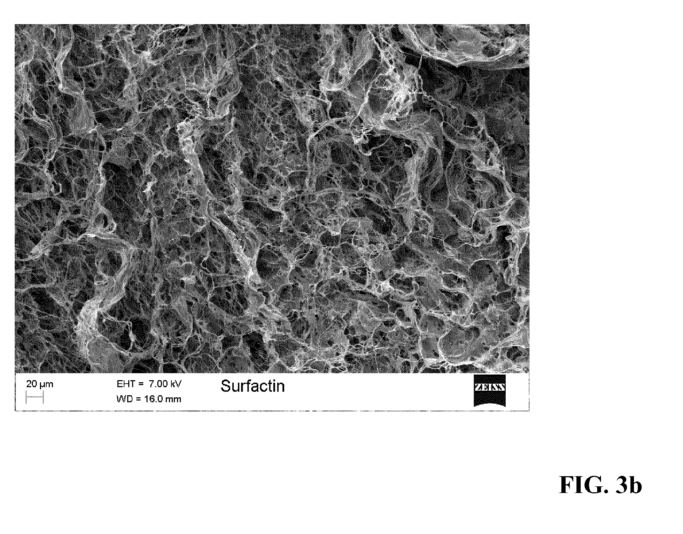

Beim Vergleich des nativen Gewebes in Figur 3a mit dem dezellularisierten Gewebe nach dem oben genannten Ausführungsbeispiels der Erfindung in Figur 3b zeigt sich eine große Ähnlichkeit der Aufnahmen. Beide Gewebe zeigen viele voneinander getrennte Kollagenfasern und -stränge.

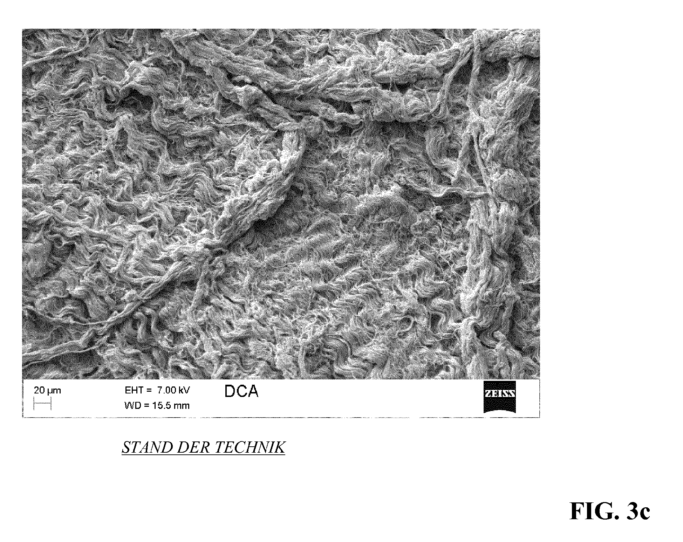

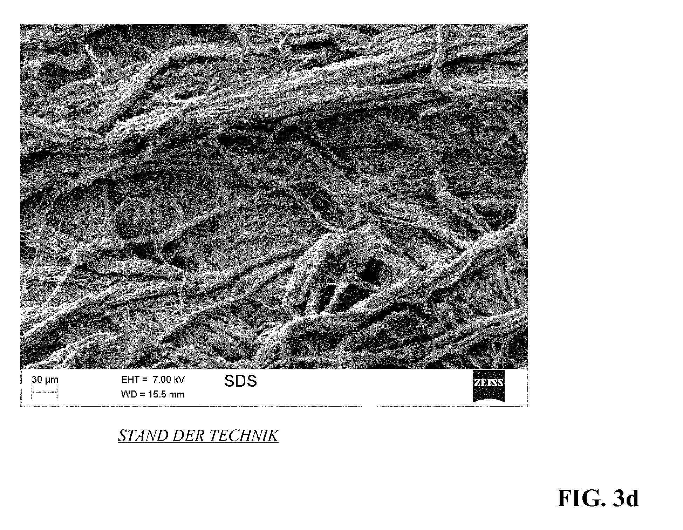

Im Vergleich dazu ist das in den Figuren 3c und 3d gezeigte Gewebe nach Dezellularisierung mit den genannten Detergenzien nach dem Stand der Technik deutlich verändert. Insbesondere kleinere Kollagenfaser neigen hier dazu, sich aneinander anzulagern. Dadurch wird die Gewebestruktur deutlich verändert und erscheint in den elektronenmikroskopischen Aufnahmen deutlich kompakter.

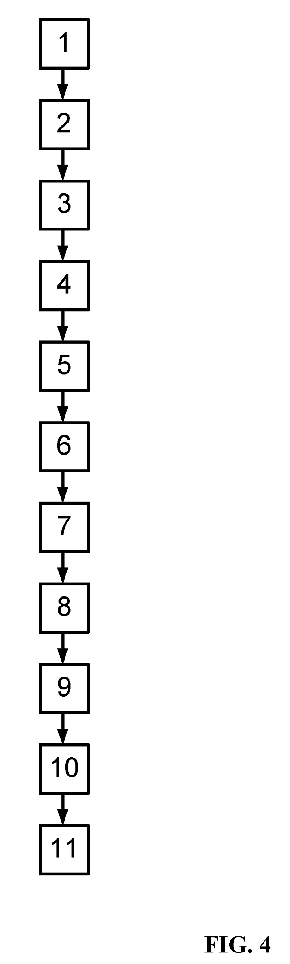

Figur 4 zeigt eine Ausgestaltung eines vollständigen Verfahrens zur Aufbereitung von biologischem Gewebe für Implantatanwendungen nach der vorliegenden Erfindung.

In Schritt 1 wird ein Herzbeutel einem Schwein im Schlachthof entnommen und für 2 Stunden bei einer Temperatur von 4°C in einer sterilen isotonischen Natriumchloridlösung (9g/l; Fa. Fresenius-Kabi) gelagert. Die Lösung enthält neben Natriumchlorid auch Penicillin und/oder Streptomycin zum Abtöten bakterieller Keime.

In Schritt 2 wird das Gewebe feucht in einer Natriumchloridlösung (9 g/l; Fa. Fresenius-Kabi) präpariert. Das heißt, hier werden die beiden Blätter des Perikards voneinander getrennt, es wird anhaftendes Fett- und Bindegewebe vorsichtig entfernt und das Gewebe in Größe und Form auf die gewünschte Anwendung zugeschnitten.

Nach dem Spülen mit einer Natriumchloridlösung (9 g/l; Fa. Fresenius-Kabi) unter leichter Bewegung des Gewebes in Schritt 3 wird das Gewebe in Schritt 4 dezellularisiert.

Die Dezellularisierung in Schritt 4 erfolgt mit einem Detergens, welches aus einer Surfactin enthaltenden Pufferlösung besteht. In diesem Ausführungsbeispiel der Erfindung wird Surfactin (Fa. Sigma-Aldrich, Surfactin from Bacillus subtilis, Art.-Nr. F3523) mit einer Konzentration von 600 mg/l in einer DPBS Phosphatpufferlösung (Fa. Lonza; DPBS w/o Ca++/Mg++; Art.-Nr. 17-512) gelöst. Das Gewebe verbleibt für 20 Stunden bei 37°C in dieser Waschlösung. Anschließend ist das Gewebe nahezu vollständig von darin befindlichen Zellbestandteilen gereinigt, ohne das dabei die Struktur der Kollagenfasern wesentlich verändert wurde.

In den darauf folgenden Schritten 5, 6 und 7 wird das dezellularisierte Gewebe ausgiebig in sterilen Lösungen gespült. In Schritt 5 wird das Gewebe in 100 ml Natriumchloridlösung (9 g/l; Fa. Fresenius-Kabi) bei Raumtemperatur unter leichter Bewegung gespült. Schritt 5 wird dabei in diesem Ausführungsbeispiel der Erfindung 10 Minuten lang durchgeführt und 8-mal wiederholt. Anschließend wird das Gewebe in Schritt 6 für 10 Minuten bei 37°C mit 100 ml einer 70%igen Ethanollösung gespült. In Schritt 7 schließt sich ein weiterer Spülschritt in 100 ml Natriumchloridlösung (9 g/l; Fa. Fresenius-Kabi) unter leichter Bewegung an.

In Schritt 8 erfolgt die Vernetzung der Kollagenfasern mit einem Vernetzungsmittel. In diesem Ausführungsbeispiel der Erfindung wird das Gewebe für 48 Stunden bei einer Temperatur von 4°C in eine Glutaraldehyd (Fa. Sigma-Aldrich, Art.-Nr. F5882) enthaltende Lösung bei pH 7.4 gelegt. Die Glutaraldehyd enthaltende Lösung besteht aus Glutaraldehyd mit einer Konzentration von 6 g/l in DPBS ohne Kalzium und Magnesium (Fa. Lonza; DPBS w/o Ca++/Mg++; Art.-Nr. 17-512).

Schritt 9 wiederholt Schritt 8 bei Raumtemperatur. Schritt 9 wird 14 Tage lang durchgeführt, wobei die Lösung aller 48 Stunden ausgetauscht wird.

Nach einem Spülprozess in Schritt 10 kann das Gewebe in Glutaraldehyd gelagert oder in Schritt 11 weiter verarbeitet werden. In Schritt 10 wird das Gewebe in diesem Ausführungsbeispiel der Erfindung 6-mal für 20 Minuten bei Raumtemperatur unter leichter Bewegung mit 100 ml Natriumchloridlösung (9 g/l; Fa. Fresenius-Kabi) gespült.

Das hier geschilderte Ausführungsbeispiel dient der Verdeutlichung der Erfindung. Zahl und/oder Ausgestaltung der Spülschritte (insbesondere Konzentrationen und Zusammensetzung der Lösung zum Spülen oder der Pufferlösung) können vom Fachmann im Rahmen seiner Kenntnisse variiert werden.

## Patentansprüche

1. Verfahren zur Aufbereitung von Gewebe für medizinische Anwendungen, insbesondere von Gewebe zur Verwendung für eine künstliche Herzklappe, wobei das Verfahren zumindest folgende Schritte umfasst:
• Dezellularisierung des Gewebes mittels eines Detergens und nachfolgende
• Vernetzung der Kollagenfasern des Gewebes mittels eines geeigneten Vernetzungsmittels,
**dadurch gekennzeichnet, dass**
das Detergens zur Dezellularisierung mindestens ein Lipopeptid mit amphiphilen Eigenschaften, bestehend aus einer hydrophilen Grundstruktur und einer hydrophoben Seitenkette, enthält.

2. Verfahren nach Anspruch 1, **dadurch gekennzeichnet, dass** das Detergens zur Dezellularisierung ein zyklisches Lipopeptid, insbesondere Surfactin, enthält.

3. Verfahren nach Anspruch 1 oder 2, **dadurch gekennzeichnet dass** das Detergens Surfactin, Daptomycin, Caspofungin, Arthrofactin , ein Echinocandin, ein Iturin, ein Syringomycin, ein Syringopeptid und/oder ein Polymyxin enthält.

4. Verfahren nach einem der vorangegangenen Ansprüche, **dadurch gekennzeichnet, dass** das Vernetzungsmittel Glutaraldehyd, Carbodiimid, Formaldehyd, Glutaraldehyd-Acetale, Acyl-Azide, Cyanimid, Genepin, Tannin, Pentagalloyl-Glukose, Phytat, Proanthocyanidin, Reuterin und/oder Epoxidverbindungen enthält.

5. Verfahren nach einem der vorangegangenen Ansprüche, **dadurch gekennzeichnet, dass** das Gewebe vor und/oder nach der Dezellularisierung zumindest einmal mit einem geeigneten Lösung, insbesondere einer Salzlösung und/oder einer Alkohollösung, gespült wird.

6. Verfahren nach einem der vorangegangenen Ansprüche, **dadurch gekennzeichnet, dass** das Detergens aus einer Pufferlösung, insbesondere einer Phosphatpufferlösung besteht, die mindestens ein Lipopeptid, insbesondere Surfactin, mit einer Konzentration von 100 mg/l bis 2000 mg/l, bevorzugt 500 mg/l bis 700 mg/l, besonders bevorzugt von 600 mg/l, enthält.

7. Verwendung einer Lösung, die mindestens ein Lipopeptid mit amphiphilen Eigenschaften, bestehend aus einer hydrophilen Grundstruktur und einer hydrophoben Seitenkette, enthält, als Detergens zur Dezellularisierung von biologischem Gewebe, insbesondere von biologischem Gewebe für Herzklappenprothesen.

8. Verwendung nach Anspruch 7, **dadurch gekennzeichnet, dass** die Lösung mindestens Surfactin, Daptomycin, Caspofungin, Arthrofactin , ein Echinocandin, ein Iturin, ein Syringomycin, ein Syringopeptid und/oder ein Polymyxin enthält.

9. Verwendung nach Anspruch 8, **dadurch gekennzeichnet, dass** Surfactin, Daptomycin, Caspofungin, Arthrofactin und/oder ein Echinocandin, Iturin, Syringomycin, Syringopeptid, Polymyxin in einer Pufferlösung, insbesondere einer Phosphatpufferlösung, gelöst ist/sind.

## Claims

1. A method for preparing tissue for medical applications, in particular tissue for use for an artificial heart valve, wherein the method has the steps, at the least, of:
• decellularizing the tissue by means of a detergent and subsequently
• cross-linking the collagen fibers of the tissue by means of a suitable cross-linking agent,
**characterized in that** the detergent for decellularization contains at least one lipopeptide having amphiphilic properties, comprising a hydrophilic base structure and a hydrophobic side chain.

2. The method according to claim 1, **characterized in that** the detergent for decellularization contains a cyclic lipopeptide, in particular surfactin.

3. The method according to claim 1 or 2, **characterized in that** the detergent contains surfactin, daptomycin, caspofungin, arthrofactin, an echinocandin, an iturin, a syringomycin, a syringopeptide, and/or a polymyxin.

4. The method according to any one of the preceding claims, **characterized in that** the cross-linking agent contains glutaraldehyde, carbodiimide, formaldehyde, glutaraldehyde acetals, acyl azides, cyanimide, genipin, tannin, pentagalloyl glucose, phytate, proanthocyanidin, reuterin and/or epoxide compounds.

5. The method according to any one of the preceding claims, **characterized in that** the tissue is rinsed before and/or after decellularization at least once with a suitable solvent, in particular a buffered saline solution and/or an alcohol solution.

6. The method according to any one of the preceding claims, **characterized in that** the detergent comprises a buffer solution, in particular a phosphate buffer solution containing at least one lipopeptide, in particular surfactin, having a concentration of 100 mg/l to 2000 mg/l, preferably 500 mg/l to 700 mg/l, particularly preferably 600 mg/l.

7. The use of a solution containing at least one lipopeptide having amphiphilic properties, comprising a hydrophilic basic structure and a hydrophobic side chain, as the detergent for the decellularization of biological tissue, in particular biological tissue for heart valve prostheses.

8. The use according to claim 7, **characterized in that** the solution contains, at least, surfactin, daptomycin, caspofungin, arthrofactin, an echinocandin, an iturin, a syringomycin, a syringopeptide, and/or a polymyxin.

9. The use according to claim 8, **characterized in that** surfactin, daptomycin, caspofungin, arthrofactin and/or an echinocandin, iturin, syringomycin, syringopeptide, polymyxin is/are dissolved in a buffer solution, in particular a phosphate buffer solution.

## Revendications

1. Procédé de traitement d'un tissu pour des applications médicales, notamment un tissu à utiliser pour une valvule cardiaque artificielle, où le procédé comprend au moins les étapes suivantes :
• la décellularisation du tissu au moyen d'un détergent, et ensuite
• une réticulation des fibres de collagène du tissu au moyen d'un agent de réticulation approprié,
**caractérisé en ce que**
le détergent pour la décellularisation contient au moins un lipopeptide avec des propriétés amphiphiles, consistant en une structure de base hydrophile et une chaîne latérale hydrophobe.

2. Procédé selon la revendication 1, **caractérisé en ce que** le détergent pour la décellularisation contient un lipopeptide cyclique, notamment de la surfactine.

3. Procédé selon les revendications 1 ou 2, **caractérisé en ce que** le détergent contient de la surfactine, de la daptomycine, de la caspofungine, de l'arthrofactine, une échinocandine, une iturine, une syringomycine, un syringopeptide et/ou une polymyxine.

4. Procédé selon l'une des revendications précédentes, **caractérisé en ce que** l'agent de réticulation contient du glutaraldéhyde, du carbodiimide, du formaldéhyde, du glutaraldéhyde-acétate, de l'azoture d'acyle, du cyanimide, de la génépine, de la tannine, du pentagalloyl-glucose, du phytate, de la proanthocyanidine, de la reutérine et/ou des composés d'époxydes.

5. Procédé selon l'une des revendications précédentes, **caractérisé en ce que** le tissu est lavé avant et/ou après la décellularisation au moins une fois avec une solution appropriée, notamment une solution saline, et/ou une solution alcoolique.

6. Procédé selon l'une des revendications précédentes, **caractérisé en ce que** le détergent est constitué par une solution tampon, notamment, une solution tampon à base de phosphate, qui contient au moins un lipopeptide, notamment, la surfactine, à une concentration de 100 mg/l à 2000 mg/l, de préférence de 500 mg/l à 700 mg/l, de manière particulièrement préférée, de 600 mg/l.

7. Utilisation d'une solution qui contient un lipopeptide, avec des propriétés amphiphiles consistant en une structure de base hydrophile et une chaîne latérale hydrophobe, en tant que détergent pour la décellularisation de tissu biologique, notamment, de tissu biologique pour des prothèse de valvules cardiaques.

8. Utilisation selon la revendication 7, **caractérisée en ce que** la solution contient au moins de la surfactine, de la daptomycine, de la caspofungine, de l'arthrofactine, une échinocandine, une iturine, une syringomycine, un syringopeptide et/ou une polymyxine.

9. Utilisation selon la revendication 8, **caractérisée en ce que** la surfactine, la daptomycine, la caspomycine, l'arthrofactine et/ou une échinocandine, une iturine, une syringomycine, un syringopeptide, une polymyxine est(sont) solubilisé(s) dans une solution tampon, notamment une solution tampon à base de phosphate.
